(19) Europäisches Patentamt
European Patent Office
Office européen des brevets

(11) Publication number: **0 211 555 A2**

(12) # EUROPEAN PATENT APPLICATION

(21) Application number: 86305556.2

(22) Date of filing: 18.07.86

(51) Int. Cl.⁴: **A61K 7/00**

(30) Priority: 01.08.85 US 761270

(43) Date of publication of application: 25.02.87 Bulletin 87/09

(84) Designated Contracting States: DE FR GB IT

(71) Applicant: DOW CORNING CORPORATION
Midland Michigan 48640(US)

(72) Inventor: Disapio, Alfred Joseph
10 Alexander Street
Greenwich Connecticut(US)
Inventor: Tassoff, James Alexander
62 Hillcrest Road
West Caldwell New Jersey(US)

(74) Representative: Lewin, John Harvey et al
Elkington and Fife High Holborn House 52/54
High Holborn
London WC1V 6SH(GB)

(54) **Transparent silicone compositions.**

(57) The addition of as little as 5% by weight of a liquid fatty alcohol or a liquid polyoxyalkylated fatty alcohol to a volatile silicone provides a composition that has useful solubilizing properties. Such a composition is particularly useful for preparing transparent personal care compositions, such as solutions, gels and sticks.

EP 0 211 555 A2

# TRANSPARENT SILICONE COMPOSITIONS

The present invention relates to compositions that contain a volatile silicone and, more particularly, to volatile silicone-containing compositions that contain a cosolvent that will dissolve the volatile silicone component and improve its compatability with other materials.

Transparent compositions have esthetic as well as functional benefits. Esthetically, transparent compositions have the appearance of being clean, pure and safe. Functionally, transparent compositions obviously allow one to see through the composition, for example to the surface of the substrate on which it has been applied. With respect to personal care compositions, the esthetics of a transparent composition frequently, but not always, are more important than the see-through quality of the composition.

Volatile silicones have found extensive use as a vehicle in various compositions and will undoubtedly continue to do so. In particular, volatile silicones are used extensively in personal care compositions such as shampoos, deodorants, antiperspirants, lotions and fragrances.

However, in most instances, the volatile silicone is not soluble in the composition and a non-transparent, e.g. translucent or opaque, composition is obtained. This is particularly true when a substantial amount, such as, for example, at least 10% by weight, of volatile silicone is incorporated into the composition.

An attempt has been made to improve the compatibility of non-volatile silicones with other materials. Thus, U.S. Patent No. 3,185,627 discloses a composition comprising a C-1 to C-3 alkyl C-8 to C-14 alkanoate and an organosilicone polymer dissolved therein. Although it is stated in the patent that the viscosity of the organosilicone polymer can have a value of from 1 to 100,000 centistokes at 25°C., volatile silicones were neither disclosed nor suggested as needing, or benefiting from, the compatibilizing effect desribed in this patent.

U.S. Patent No. 4,346,079 discloses the use of a silicone oil, as well as other oleaginous materials, in a transparent gelled antiperspirant stick to reduce the tackiness of the stick. However, the amount of silicone oil that is used in the stick is so small that an incompatibility problem with its use in the stick would not be expected. In addition, although cyclic polydimethylsiloxanes are suggested as a suitable silicone oil in the compositions of this patent there is no specific examples that show the use thereof.

It is an object of this invention to provide transparent compositions which contain a volatile silicone and one or more components which are normally incompatible with a volatile silicone, i.e. which normally do not form a transparent mixture or solution with a volatile silicone. It is another object of this invention to provide a transparent volatile silicone-containing composition which can be mixed with one or more personal care components to provide a transparent personal care composition.

These objects, and other which will be apparent to one skilled in the silicone formulating art on considering the present disclosure and appended claims, are obtained by the present invention which, briefly stated, comprises incorporating into a volatile silicone a cosolvent component which is compatible with the volatile silicone and which will compatibilize the volatile silicone with other materials.

It has been discovered that when the cosolvent component is a liquid fatty alcohol and/or a liquid polyoxyalkylated fatty alcohol the resulting mixture has unusual solvating effects.

The present invention relates to a transparent composition comprising at least 10% by weight, based on the weight of the composition, of a volatile silicone, and at least 5% by weight based on the weight of the volatile silicone, of a cosolvent selected from the group consisting of liquid fatty alcohols and liquid polyoxyalkylated fatty alcohols.

By the term volatile silicone, it is meant a polydiorganosiloxane having normal boiling point of less than 250°C. Examples of volatile silicones that are suitable for use in this invention include cyclopolydimethylsiloxanes having the formula -$(Me_2SiO)_x$ wherein x denotes 3, 4, 5 or 6 and methyl-terminated linear polydimethylsiloxanes having the formula $Me(Me_2SiO)_ySiMe_3$ wherein y has a value of 1, 2, 3, or 4.

For personal care compositions, the volatile silicone component of the compositions of this invention is preferably a cyclopolydimethylsiloxane having from four to six silicon atoms per molecule.

The cosolvent component can be a liquid fatty alcohol. Liquid fatty alcohols are well known, particularly in the personal care formulating art, and need no detailed delineation herein. Fatty alcohols that are suitable for use in the compositions of the present invention are liquid, i.e. pourable at room temperatures and contain one or more carbon-bonded hydroxyl radicals. The carbon skeleton of the fatty alcohol can be linear, cyclic or branched and saturated or unsaturated.

Examples of suitably liquid fatty alcohols include, but are not limited to, lauryl alcohol, isostearyl alcohol, isocetyl alcohol, oleyl alcohol, linoleyl alcohol and linolenyl alcohol.

The cosolvent component can be a liquid polyoxyalkylated fatty alcohol. That is to say, the cosolvent component can be a polyoxyalkylated liquid or solid fatty alcohol provided that it is a liquid at room temperature. The polyoxyalkylene portion can contain polyoxyethylene and/or polyoxypropylene units; however, for a preferred solvating effect the polyoxyalkylated portion of the polyoxyalkylated fatty alcohol preferably contains polyoxypropylene units and, most preferably, only polyoxypropylene units.

Examples of suitably liquid polyoxyalkylated fatty alcohols include, but are not limited to, the various polyoxypropylated stearyl, cetyl, myristyl, lauryl, and oleyl alcohols and their mixtures. Specific examples thereof include, but are not limited to, PPG-10 Cetyl Ether, PPG-15 Stearyl Ether, PPG-30 Stearyl Ether, PPG-50 Stearyl Ether, and PPG-5-PEG-20 Cetyl Ether. In these examples of polyoxyalkylated fatty alcohols, PPG and PRG denote polypropylene glycol units and polyethylene glycol units, respectively, and the number following PPG or PEG denotes the number of oxyalkylene units that are present in the polyoxylakylene portion of the ether molecule.

The cosolvent component can also be a mixture of one or more liquid fatty alcohols and one or more liquid polyoxyalkylated fatty alcohols, if desired. A preferred cosolvent mixture is a mixture of isostearyl alcohol and polyoxypropylated cetyl alcohol.

The compositions of the present invention can consist essentially of the volatile silicone component plus the liquid cosolvent. In this case, the transparent mixture can be used to formulate transparent compositions, particularly compositions that would not be transparent if formulated only with the volatile silicone component.

The compositions of the present invention can also contain, in addition to the volatile silicone component and the liquid cosolvent component, one or more additional components which are commonly used in personal care compositions, such as skin care components, hair care components, hygiene components and cosmetic components.

Examples of skin care components which are suitable components in the compositions of this invention include, but are not limited to, skin-conditioning components, such as humectants, moisturizers and emollients; skin-protecting components, such as sunscreens, barrier oils and resins and insect repellants; and topical medicaments, such as steroids, vitamins, anesthetics, antibiotics and penetrants.

Examples of hair care components which are suitable components in the composition of this invention include, but are not limited to, detergents, conditioners, curling lotions, hair-straightening lotions, bleaches and dyes.

Examples of hygiene components which are suitable components in the composition of this invention include, but are not limited to, antiseptics, deodorants and antiperspirants.

Examples of cosmetic components which are suitable components in the composition of this invention include, but are not limited to, colorants, fragrances and bleaches.

Examples of additional components which are suitable components in the compositions of this invention include, but are not limited to, stick-forming components such as sodium stearate, waxes, esters of fatty acids, fatty esters of carboxylic acids and alcohols such as ethanol, propylene glycol and isopropyl alcohol.

The relative amounts of the volatile silicone component and the liquid cosolvent component that are to be used in the compositions of this invention are not narrowly limited. For example, as little as 5 parts of cosolvent for every 100 parts of volatile silicone is expected to provide a composition having significantly improved solvating, i.e., compatibilizing, ability, compared to the volatile silicone alone. On the other hand, as little as 5 parts of volatile silicone for every 100 parts of cosolvent is expected to provide a composition having the efficacious action of the volatile silicone and the solvating ability of the cosolvent.

A preferred composition of this invention comprises at least one part of volatile silicone for every two parts of liquid cosolvent, i.e., the weight ratio of the latter to the former has a value of no more that 2 and preferably no more than 1.

The following examples are disclosed to further show how to practice, but not to limit, this invention. All parts and percentages are by weight unless stated otherwise.

The term transparent, as used herein, denotes the condition which allows objects to be seen distinctly through a geometrically suitable body having a thickness of 25 millimeters. In the present invention, the term body refers to a solid composition of this invention or a liquid composition of this invention contained in a suitable container.

GLOSSARY:

$D_4$; Octamethylcyclotetrasiloxane, $[(CH_3)_2SiO]_4$.

$D_5$; Decamethylcyclopentasiloxane, $[(CH_3)_2SiO]_5$.

PPG-10 Cetyl Ether; $CH_3(CH_2)_{14}CH_2(OCH(CH_3)CH_2)_nOH$

where n has an average value of 10.

PPG-3 Myristyl Ether; $CH_3(CH_2)_{12}CH_2(OCH(CH_3)CH_2)_nOH$

where n has an average value of 3.

PPG-5-Ceteth-20; $CH_3(CH_2)_{14}CH_2(OCH(CH_3)CH_2)_x(OCH_2CH_2)_yOH$

where x has an average value of 5 and y has an average value of 20.

PPG-15 Stearyl Ether; $CH_3(CH_2)_{16}CH_2(OCH(CH3)CH_2)_nOH$

where n has an average value of 15.

PPG-30 Stearyl Ether; ditto, n has an average value of 30.

PPG-50 Stearyl Ether; ditto, n has an average value of 50.

EXAMPLE 1

Seven mixtures consisting of one part stearyl alcohol (a solid fatty alcohol; not compatible with $D_4$) and 99 parts of a mixture of isostearyl alcohol and $D_4$ were prepared by mixing suitable amounts of the three components. The weight ratio of $D_4$ to isostearyl alcohol had values of 90.67/8.33, 87.33/11.67, 84/15. 80.67/18/33, 77.33/21.67, 74/25 and 67.7/31.3. While the amount of stearyl alcohol precipitate varied inversely with amount of isostearyl alcohol that was used, only the 67.7/31.3 composition was transparent.

EXAMPLE 2

The experiments of Example 1 were repeated using liquid fatty alcohols other than isostearyl alcohol and at three ratios of $D_4$ to liquid fatty alcohol; i.e., 94.05/4.95, 87.45/11.55 and 80.85/18.15. Transparent compositions were obtained at ratios of 80.85/18.15 for oleyl alcohol and isocetyl alcohol and at ratios of 87.45/11.55 and 80.85/18.15 for a liquid mixture of straight-chain C-12 to C-14 fatty alcohols.

EXAMPLE 3

A transparent stick was prepared by mixing, at elevated temperature, 14.9 parts of propylene glycol, 7.5 parts of ethanol, 10 parts of PPG-10 Cetyl Ether, 19.8 parts of isostearyl alcohol and 39.8 parts of $D_5$. Eight parts of sodium stearate powder were added to and dissolved in the hot mixture using high shear. On cooling, the above composition solidified to a transparent stick. It will be obvious to those skilled in the personal care formulation art that additional personal care components, such as a deodorant or a fragrance can be added to the mixture before it solidifies, if desired.

EXAMPLE 4

A transparent medicament solution was prepared by mixing 6.1 parts of benzocaine 43.9 parts of PPG-15 Stearyl Ether 13.6 parts of PPG-10 Cetyl Ether and 36.4 parts of $D_4$. In the absence of the polyoxypropylated fatty alcohols, the mixture of benzocaine and $D_4$ was not transparent.

EXAMPLE 5

When an incompatible mixture of 1 part fragrance oil and 100 parts of $D_4$ is mixed with 3 parts of oleyl alcohol or isostrearyl alcohol, the mixture becomes transparent. Examples of said fragrance oils are cinnamon bark oil, phenyl alcohol, olibanum resinoid, ylang ylang oil, anisic alcohol, cinnamyl alcohol and hydroxy citronellal.

EXAMPLE 6

A transparent medicament stick was prepared by mixing, at elevated temperature, 23.5 parts of $D_4$, 9.4 parts of PPG-10 Cetyl Ether, 32.9 parts of PPG-15 Stearyl Ether, 23.5 parts of propylenen glycol and 6 parts of benzocaine. Sodium stearate, 4.7 parts, was added to an dissolved in the hot mixture. On cooling, this composition solidified to a transparent stick.

**Claims**

1. A transparent composition containing at least 10% by weight, based on the weight of the composition, of a volatile silicone, and at least 5% by weight, based on the weight of the volatile silicone, of a cosolvent selected from the group consisting of liquid fatty alcohols and liquid polyoxyalkylated fatty alcohols.

2. A transparent composition according to claim 1 wherein the volatile silicone is a cyclopolydimethylsiloxane.

3. A transparent composition according to claim 1 or 2 further comprising one or more personal care components.

4. A transparent composition according to any of claims 1 to 3 wherein the liquid fatty alcohol is selected from the group consisting of oleyl alcohol and isostearyl alcohol.

5. A transparent composition according to any of claims 1 to 4 wherein the weight ratio of the cosolvent to the volatile silicone has a value of no more than 2.

6. A transparent composition according to any of claims 1 to 5 wherein the liquid polyoxyalkylated fatty alcohol is selected from the group consisting of polyoxypropylated stearyl alcohol, polyoxyethylated derivatives thereof, polyoxypropylated cetyl alcohol and poloxyethylated derivatives therof.

7. A transparent composition according to any of claims 1 to 6 consisting essentially of cyclopolydimethylsiloxane having from 4 to 6 silicon atoms and a mixture of isostearyl alcohol and polyoxypropylated cetyl alcohol.